# EUROPEAN PATENT SPECIFICATION

(11) **EP 0 262 798 B1**
(45) Date of publication and mention of the grant of the patent: **18.05.1994**
(21) Application number: 87307660.8
(22) Date of filing: 28.08.1987
(51) Int. Cl.: A61N 1/08, A61N 1/44, H01C 1/024

(54) **Output controller for electrotherapeutic device directed to use in bath**
Ausgangssteuerung für eine elektrotherapeutische Einrichtung zum Gebrauch beim Baden
Contrôleur de sortie pour un dispositif électrothérapeutique pour l'utilisation dans le bain

(30) Priority: 31.08.1986 JP 133327/86
(43) Date of publication of application: 06.04.1988
(73) Proprietor: Hayashibara, Ken, Okayama-shi Okayama (JP)
(72) Inventor: Masaki, Kazumi, Suita-shi Osaka (JP)
(74) Representative: Jenkins, Peter David

(56) References cited:
- DE-A- 1 548 783
- DE-A- 1 665 787
- DE-A- 2 262 385
- DE-A- 2 721 757
- DE-C- 657 458
- FR-C- 811 113
- US-A- 2 862 088
- US-A- 2 862 089
- US-A- 2 917 720
- US-A- 2 917 721
- US-A- 3 184 697
- Siegfried Hildebrand, "Feinmechanische Bauelemente", 4th edition, Carl Hanser Verlag München Wien, 1983, page 722

## Description

The present invention relates to an output controller for low-frequency electrotherapeutic device directed to use in bath.

Conventional devices require protection from water and moisture when used in a bath or bath water. Although the low-frequency oscillator and battery can be protected by enclosing them in a container having sufficient moistureproof and water-resistive properties, perfect protection of the variable resistor and power switch is very difficult because they may have a movable part such as a rotary shaft. Reference is made in this respect to DE-A-2.262.385 which describes a low-frequency electrotherapeutic device which has no water resistive protection.

In DE-A-2.721.757 a low-frequency medical treatment apparatus is described. This document addresses the problems in preventing excessive electrical power being applied to the human body and describes a shock preventing circuit.

In "Feinmechanische Bauelemente" - Siegfried Hildebrand, 4th ed, the protection of precision mechanical components from mechanical, electrical, magnetic and climatic effects is described. In particular, the standard International Protection Code letters are explained and some basic sealing structures described for rotary shafts through walls where the sealing member is located between the shaft and the wall of the hole through which the shaft passes.

Reference is also made to DE-C-0.657.458 and FR-C-0.811.113 which both describe switches sealed by means of a plate-like resilient material in which the switches are movable to describe an arc about a fulcrum located behind the resilient material.

It is an aim of the present invention to provide an output controller for a low-frequency electrotherapeutic device wherein the rotary shaft of the variable resistor is elastically and airtightly held in order to prohibit the permeation of humidity and moisture through the movable parts.

The present invention accordingly provides an electrotherapeutic device using a variable resistor for control thereof, comprising: a container having a water-proof and moisture-proof structure; an oscillator that generates a low-frequency therapeutic voltage, said oscillator being enclosed in said container; and said variable resistor comprising: a variable resistor having a rotary shaft, said variable resistor being connected with an output terminal of said oscillator to control the output level thereof and also being enclosed in said container in such a manner that an end of said rotary shaft projects outwardly through said container characterised in that there is further provided means to hermetically seal a clearance between said rotary shaft and container, said sealing means comprising: a soft resilient material in sheet or plate shape that covers a top outside wall of the container, said soft resilient material having a hole in which the rotary shaft is hermetically and rotatably inserted; and a mounting member being removably attached to an outside wall of said container such that a peripheral part of said soft resilient material is pressed onto the top outside wall of said container.

An embodiment of the present invention will now be described by way of example only with reference to the accompanying drawings, in which:
FIG. 1 is a vertical sectional view of an electrotherapeutic device wherein the bearing is composed of a soft resilient material;
FIG. 2 is a vertical sectional view of the principle part of an embodiment according to the present invention wherein a sealing member of a soft resilientmaterial is provided; and
FIG. 3 is a vertical sectional view of the principal part of a therapeutic device using a spring.

In the drawings, reference numeral (1) designates container; (2), low-frequency oscillator including amplifier; (3), battery; (4), variable resistor; (5), rotary shaft; (6), bearing portion; (7), knob; (8), lock nut; (9), sealing member; (10), periphery of the sealing member; (11), mounting member; (12), bore provided through the sealing member; (13), tapered bore in bearing; (14), tapered plug; (15), spring; and (16), pin.

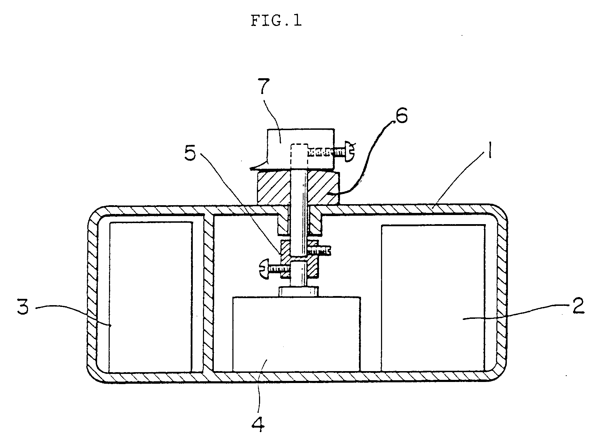

FIG. 1 is the vertical sectional view of an electrotherapeutic device not in accordance with the invention wherein a bearing is provided and is composed of a soft resilient material. Reference numeral (1) designates the container of a waterproof and moistureproof structure for enclosing battery (3) and low-frequency oscillator (2) including amplifier. Reference numeral (4) designates a variable resistor that is built in container (1) to control the output level of low-frequency oscillator (2). Reference numeral (5) designates the rotary shaft of variable resistor (4), and a part of rotary shaft (5) projects outwardly through container (1). Rotary shaft (5) is rotatably held in bearing (6) provided through container (1). Although desirably rotary shaft (5) and variable resistor (4) are provided in one body, a prescribed shaft length can be attained by connecting an axis member with an appropriate length to rotary shaft (5) as shown in FIG. 1. Bearing (6) is composed of a soft material resilient enough to elastically, airtightly and rotatably hold the periphery of rotary shaft (5), such as rubber and synthetic resin. Thus, permeation of humidity and moisture through the bearing clearance can be prevented. Reference numeral (7) designates a knob provided at the end of rotary shaft (5), and rotary shaft (5) rotates in association with knob (7). Knob (7) can be suitably marked in correspondence with a dial that may be provided on container (1).

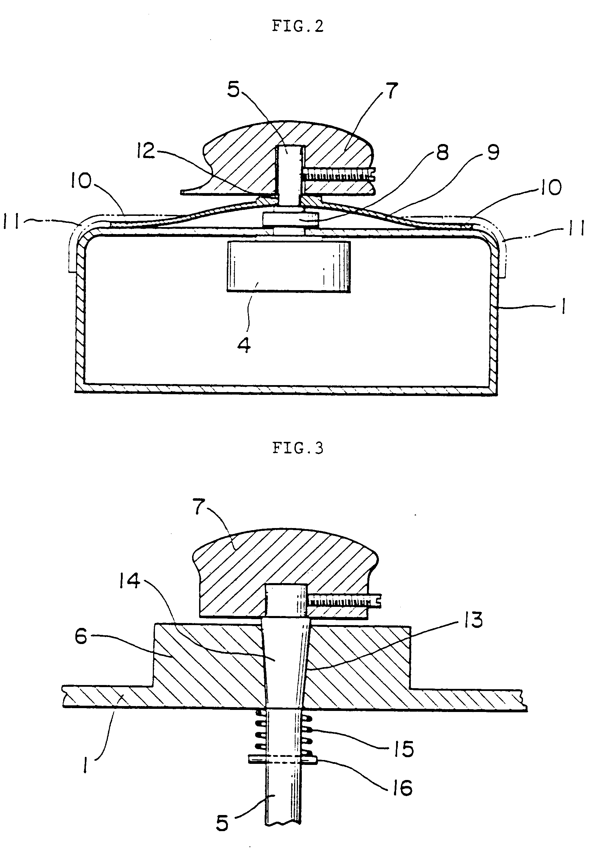

FIG. 2 is the vertical sectional view of the principal part of an embodiment according to the invention wherein a sealing member of a soft resilient material is provided. Variable resistor (4) is attached to container (1) with lock nut (8) so that rotary shaft (5) projects outwardly through container (1). Reference numeral (9) designates a sealing member of a soft resilient material in sheet or plate shape, such as rubber or synthetic resin, that is arranged to cover the upper part of container (1). Reference numeral (10) designates the periphery of sealing member (9) that is adhered on the upper surface of container (1). To attain such adhesion, beside using an adhesive, a method wherein mounting member (11) removably provided by screwing around the outside wall of container (1) pushes and fixes rotary shaft (5) can be employed. Reference numeral (12) designates a bore provided centrically through sealing member (9) in which the periphery of rotary shaft (5) is elastically and airtightly held to prevent possible permeation of humidity and moisture through the bearing clearance.

FIG. 3 is the vertical sectional view of the principal part of another electrotherapeutic device not in accordance with the invention using a spring. In this Figure, bore (13) of bearing (6) is tapered, while plug member (14) correspondently tapered is provided as the end of rotary shaft (5). These tapers can be upwardly or downwardly provided. Reference numeral (15) designates a spring to be placed between bearing portion (6) and pin (16) provided on rotary shaft (5). When the taper is provided downwardly, spring (15) is placed between bearing (6) and knob (7) to be attached to rotary shaft (5).

Since airtight insertion of plug (14) into bearing (13) by spring (15) elastically and airtightly seal the clearance therebetween, permeation of humidity and water through bore (13) can be effectively prohibited. The effect is further improved by using bearing (6) composed of a soft resilient material such as rubber or synthetic resin.

Since the present invention is arranged using a soft resilient material in sheet or plate form to cover the top outside wall of the container, it is very useful as the output controller for low-frequency therapeutic device directed to use in bath or bath water.

Furthermore, the use of a low-frequency therapeutic device according to the invention is effective in improvement and treatment of blood circulation, muscular strength, muscular fatigue, and haemorrhoids. At a frequency of 100 hertz or lower, preferably, about 10-70 hertz, the device is effective in improvement and treatment of blood circulation, muscular strength, muscular fatigue and haemorrhoids; and at a frequency of 100-300 hertz, in prevention of alopecia as well as acceleration and regeneration of hair.

## Claims

1. An electrotherapeutic device using a variable resistor (4) for control thereof, comprising:
a container (1) having a water-proof and moisture-proof structure;
an oscillator (2) that generates a low-frequency therapeutic voltage, said oscillator (2) being enclosed in said container (1); and
said variable resistor (4) comprising:
a variable resistor (4) having a rotary shaft (5), said variable resistor (4) being connected with an output terminal of said oscillator (2) to control the output level thereof and also being enclosed in said container (1) in such a manner that an end of said rotary shaft (5) projects outwardly through said container (1) characterised in that there is further provided
means to hermetically seal a clearance between said rotary shaft (5) and container (1), said sealing means comprising:
a soft resilient material (9) in sheet or plate shape that covers a top outside wall of the container (1), said soft resilient material (9) having a hole in which the rotary shaft (5) is hermetically and rotatably inserted; and
a mounting member (11) being removably attached to an outside wall of said container such that a peripheral part of said soft resilient material (9) is pressed onto the top outside wall of said container (1).

2. The device of claim 1, wherein said soft resilient material is of rubber or synthetic resin.

3. The device of claim 1, wherein the frequency of said low-frequency voltage is in the range of 10-70 hertz or of 100-300 hertz.

## Patentansprüche

1. Elektrotherapeutisches Gerät mit einem veränderlichen Widerstand (4) zur Steuerung desselben, welches aufweist: einen Behälter (1) mit einem wasserdichten und feuchtigkeitsdichten Aufbau;
einen Oszillator (2), der eine niederfrequente therapeutische Spannung erzeugt, wobei der Oszillator (2) in dem Behälter (1) eingeschlossen ist; und
wobei der veränderliche Widerstand (4) besteht aus:
einem veränderlichen Widerstand (4) mit einer drehbaren Welle (5), wobei der veränderliche Widerstand (4) mit einer Ausgangsklemme des Oszillators (2) verbunden ist, um den Ausgangswert desselben zu steuern, und ebenfalls in dem Behälter (1) derart eingeschlossen ist, daß ein Ende der drehbaren Welle (5) durch den Behälter (1) nach außen ragt,
dadurch gekennzeichnet, daß ferner vorgesehen ist
eine Einrichtung zur hermetischen Abdichtung eines Spaltes zwischen der drehbaren Welle (5) und dem Behälter (1), wobei die Abdichteinrichtung besteht aus:
einem weichelastischen Material (9) in Folien- oder Plattenform, das eine obere Außenwand des Behälters (1) überdeckt, wobei das weichelastische Material (9) ein Loch aufweist, in das die drehbare Welle (5) hermetisch dicht und drehbar eingesetzt ist; und
einem Halteteil (11), der abnehmbar an einer Außenwand des Behälters derart befestigt ist, daß ein Umfangsteil des weichelastischen Materials (9) auf die obere Außenwand des Behälters (1) gedrückt wird.

2. Gerät nach Anspruch 1, bei welchem das weichelastische Material aus Gummi oder Kunstharz besteht.

3. Gerät nach Anspruch 1, bei welchem die Frequenz der niederfrequenten Spannung im Bereich von 10 - 70 Hz oder von 100 - 300 Hz liegt.

## Revendications

1. Dispositif électrothérapeutique utilisant une résistance variable (4) pour sa commande, comprenant :
- un conteneur (1) ayant une structure étanche à l'eau et étanche à l'humidité ;
- un oscillateur (2) qui produit une tension thérapeutique de basse fréquence, ledit oscillateur (2) étant enfermé dans ledit conteneur (1) ; et
ladite résistance variable (4) comprenant :
- une résistance variable (4) comportant un arbre de rotation (5), ladite résistance variable (4) étant reliée à une borne de sortie dudit oscillateur (2) pour en commander le niveau de sortie et étant aussi enfermée dans ledit conteneur (1) d'une manière telle qu'une extrémité dudit arbre de rotation (5) se prolonge vers l'extérieur à travers ledit conteneur (1), caractérisé en ce qu'il est prévu en outre
- un moyen pour sceller hermétiquement un espacement entre ledit arbre de rotation (5) et ledit conteneur (1), ledit moyen d'étanchéité comprenant:
- de la matière élastique molle (9) en forme de feuille ou de plaque qui recouvre la paroi supérieure extérieure du conteneur (1), ladite matière élastique molle (9) comportant un trou dans lequel l'arbre de rotation (5) est introduit de façon hermétique et pour pouvoir tourner ; et
- un élément de montage (11) fixé, de façon à pouvoir être enlevé, à une paroi extérieure dudit conteneur de sorte qu'une partie périphérique de ladite matière élastique molle (9) est comprimée sur la paroi supérieure extérieure dudit conteneur (1).

2. Dispositif selon la revendication 1, dans lequel ladite matière élastique molle est du caoutchouc ou de la résine synthétique.

3. Dispositif selon la revendication 1, dans lequel la fréquence de ladite tension de basse fréquence est située dans une plage de 10 à 70 hertz ou dans une plage de 100 à 300 hertz.
